(19) Europäisches Patentamt
European Patent Office
Office européen des brevets

(11) Publication number: 0 254 577 B1

(12) # EUROPEAN PATENT SPECIFICATION

(45) Date of publication of the patent specification: 27.09.89

(51) Int. Cl.⁴: C07D 311/74, C07D 307/79, C07D 317/48, C07D 319/18, C07D 263/56, C07D 265/36

(21) Application number: 87306538.7

(22) Date of filing: 23.07.87

(54) **Synthesis of bicyclic aromatic sulfonyl chlorides.**

(30) Priority: 23.07.86 US 889141

(43) Date of publication of application: 27.01.88 Bulletin 88/4

(45) Publication of the grant of the patent: 27.09.89 Bulletin 89/39

(84) Designated Contracting States: AT BE CH DE ES FR GB GR IT LI NL SE

(56) References cited:
EP-A- 0 187 387

CHEMICAL ABSTRACTS, vol. 89, no. 13, September 25 1978, Columbus, Ohio, USA: PRAJAPATI S.P.; PARDANANI J.H.; SETHNA S. "Studies on 4-4'-dihydroxydiphenyl sulfone and 4,4'-dihydroxydiphenyl ether" page 867, column 2, abstract-no. 108953q.
CHEMICAL ABSTRACTS, vol. 87, no. 15, October 10, 1977, Columbus, Ohio, USA: OKAMOTO S; KIKUMOTO R; TAMAO Y; OKUBO K; TEZUKA T; TONOMURA S; HIJIKATA A; "NG-Protected N2-(heterocyclesulfonyl) argininamides" page 625, column 2, abstract-no. 118068d.
Blodgett-Technik"121-30 1983, 52(1), 115-19 000
CHEMICAL ABSTRACTS, vol 88, no 3, Januarz 16, 1978, Columbus, Ohio, USA: OKAMOTO S; KIKUMOTO Y;

(73) Proprietor: **ELI LILLY AND COMPANY, Lilly Corporate Center, Indianapolis Indiana 46285(US)**

(72) Inventor: **Aikins, James Abraham, 7731 Hallow Ridge Circle, Indianapolis Indiana 46256(US)**
Inventor: **Tao, Eddie Vi-Ping, 1211 Kirkham Lane, Indianapolis Indiana 46260(US)**

(74) Representative: **Hudson, Christopher Mark et al, Erl Wood Manor, Windlesham Surrey GU20 6PH(GB)**

(56) References cited: (continuation)
OKUBO K; TEZUKA T; TONOMURA S; HIJIKATA A; "NG-Substituted-n2-substituted argininamides" page 685, column 2, abstract-no. 23390h.

EP 0 254 577 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention provides a novel process for preparing bicyclic aromatic sulfonyl chlorides.

Benzene sulfonylureas of the formula

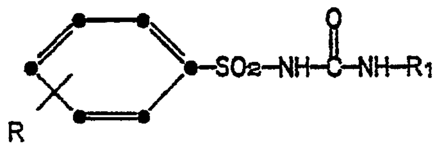

where R is an organic radical, are well known in the art. Several methods of preparing such compounds are known and are generally summarized by Kurzer, Chem. Rev., 50, 1 (1952). One synthesis involves reacting a substituted benzene sulfonamide of the formula

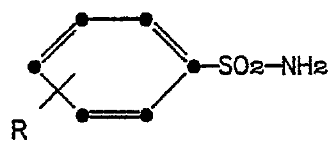

with an isocyanate of the formula

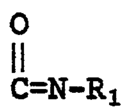
.

In many cases the required sulfonamide is not commercially available and must be synthesized. Such compounds are routinely prepared by synthesizing the sulfonyl chloride derivative, followed by ammonolysis with ammonium hydroxide or ammonia.

Several methods of synthesizing sulfonyl chlorides are taught in the art. Shah et al., J. Med. Chem., 12, 938 (1969) describe a procedure for preparing sulfonyl chlorides known as the Meerwein Procedure. The Meerwein Procedure involves diazotization of an aniline, followed by reaction with a solution of cupric chloride, concentrated hydrochloric acid, and sulfur dioxide in glacial acetic acid.

Breuer et al., Chimie. Therapeutique, 659 (1979) describe two methods for synthesizing bicyclic aromatic sulfonyl chlorides. The first method involves a high temperature sulfonation of bicyclic aromatic compounds using hydrogen sulfate, followed by chlorination of the intermediate sulfonic acid. The second method involves a low temperature chlorosulfonation employing chlorosulfonic acid to both sulfonate and chlorinate.

Breuer et al. also synthesized an oxygen containing bicyclic aromatic sulfonamide; dihydrobenzofuran-6-sulfonamide. The synthetic route disclosed utilized the diazonium salt of 3-amino-4-(β-chloroethyl)benzene sulfonamide and did not require the synthesis of the corresponding sulfonyl chloride.

Unfortunately, it is not possible to synthesize oxygen containing bicyclic aromatic sulfonyl chlorides by the procedures known in the art. Both the Meerwein Procedure of Shah et al. and the chlorosulfonation method of Breuer et al. cannot be used to prepare oxygen containing bicyclic aromatic sulfonyl chlorides since the conditions employed destroy the oxygen containing ring.

According to the present invention there is provided a process for preparing bicyclic aromatic sulfonyl chlorides of the formula

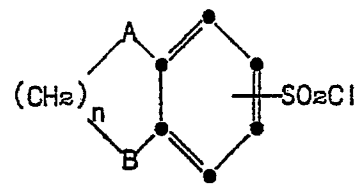

wherein, n is 1 or 2;
A is -O- or $-CH_2-$; and
B is -O-, $-CH_2-$, or $-NCH_3-$;
provided at least one of A or B is -O-,
which comprises reacting a compound of the formula

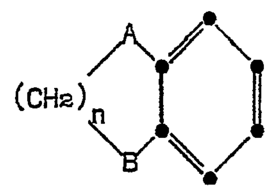

with a Villsmeier reagent of the formula

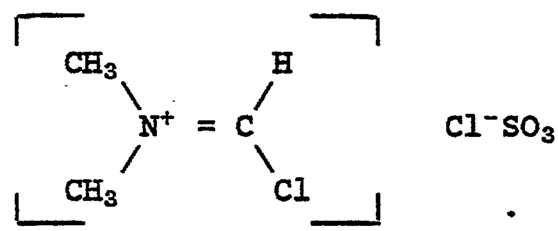
.

The process of the invention provides a method of synthesizing oxygen containing bicyclic aromatic sulfonyl chlorides since the conditions employed will not destroy the oxygen containing ring.

The present invention relates to a process for preparing bicyclic aromatic sulfonyl chlorides which are useful as intermediates in the formation of sulfonylureas. In the process of the invention, a bicyclic aromatic compound is reacted with a Villsmeier reagent prepared by reacting approximately equimolar amounts of sulfuryl chloride and dimethylformamide (DMF). While the bicyclic compound and the Villsmeier reagent generally are employed in approximately equimolar quantities, the Villsmeier reagent can be added in up to about a twenty percent molar excess. The chlorosulfonation reaction is run at temperatures of about 50°C to about 135°C, with the most desired temperature of about 75°C to about 125°C. The reaction generally is substantially complete after about five to ninety minutes, but longer reaction periods are not detrimental.

The resulting sulfonyl chloride can be isolated, if desired, but need not be, and is readily converted to a sulfonamide by ammonolysis with ammonium hydroxide or ammonia according to well known techniques. After the sulfonamide is formed, it can be reacted with an isocyanate to yield a sulfonylurea, which compounds are well known in the art. In general, sulfonylureas are taught to have oral hypoglycemic activity. In addition, some antimycotic activity is noted and the compounds have also been prepared as derivatives of carbodiimides.

The starting materials for the Villsmeier reagent, DMF and sulfuryl chloride, are commercially available. The bicyclic aromatic starting materials are either commercially available, known in the literature, or can be prepared by methods known in the art.

The most preferred compounds which can be made by the process of this invention are, when n is 1, 1,3-benzodioxole-5-sulfonyl chloride and dihydrobenzofuran-5-sulfonyl chloride, and, when n is 2, 1,4-benzodioxan-6-sulfonyl chloride.

The following examples illustrate the process of this invention. The examples are illustrative only and are not intended to limit the scope of the invention in any way.

Example 1

1,3-Benzodioxole-5-sulfonamide

A 500 ml 3-neck round bottom flask was charged with 38.7 g (0.52 mol) of dimethylformamide. The contents of the flask were cooled to 0°C and diluted by addition of 70.18 g (0.52 mol) of sulfuryl chloride at a rate such that the temperature of the reaction mixture remained below 20°C. Following complete addition, the reaction mixture was stirred for 10 minutes while maintaining the temperature at approximately 10°C.

After the Villsmeier reagent was formed, 61.06 g (0.5 mol) of 1,3-benzodioxole were added dropwise over a period of 5 minutes. The mixture was stirred and heated to 80°C for approximately 10 minutes, and then at 110°C for another 5 minutes. During the reaction the solution turned reddish brown.

After about 15 minutes, the reaction mixture was cooled to 40°C and poured into a mixture of 450 g crushed ice, 200 ml water, and 200 ml of chloroform. The organic layer was separated and added dropwise to 200 ml of concentrated ammonium hydroxide. The alkaline solution was stirred for approximately ninety minutes. The organic and aqueous phases were allowed to separate and a yellow granular precipitate formed at the interface of the two layers.

This solid was collected by filtration and washed with 100 ml of water. The resulting solid was dried overnight at 40°C to provide 26.9 g of 1,3-benzodioxole-5-sulfonamide (yield: 26.7%). The product melted at 158-160°C. Both mass spectroscopy and n.m.r. assays indicated the product was 1,3-benzodioxole-5-sulfonamide.

Example 2

1,4-Benzodioxan-6-sulfonamide

A 500 ml 3-neck round bottom flask was charged with 27.78 g (0.38 mol) of dimethylformamide. The contents of the flask were cooled to 0°C and diluted by addition of 51.3 g (0.38 mol) of sulfuryl chloride at a rate such that the temperature of the reaction mixture was maintained below about 20°C. Following complete addition, the reaction mixture was stirred for 10 minutes while maintaining the temperature at approximately 10°C.

After forming the Villsmeier reagent 50.0 g (0.36 mol) of 1,4-benzodioxan were added dropwise over a period of 5 minutes. The reaction mixture was stirred and heated to 125°C for about seventy-five minutes.

The reaction mixture was cooled to 40°C and poured into a mixture of 500 g crushed ice and 200 ml chloroform. The organic layer was separated and added dropwise to a mixture of 450 ml concentrated ammonium hydroxide and 200 ml of water. The alkaline solution was stirred overnight at 25°C. The organic and aqueous phases were allowed to separate and a yellow granular precipitate formed at the interface of the two layers.

This solid was recovered, washed, and dried per the procedure of Example 1 to provide 26.3 g of 1,4-benzodioxan-6-sulfonamide (yield: 34.0%). The product solid melted at 196-198°C. Both mass spectroscopy and n.m.r. assays indicated the product was 1,4-benzodioxan-6-sulfonamide.

## Claims

1. A process for preparing bicyclic aromatic sulfonyl chlorides of the formula

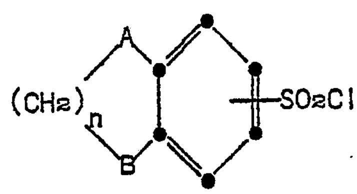

wherein,
n is 1 or 2;
A is -O- or $-CH_2-$; and
B is -O-, $-CH_2-$, or $-NCH_3-$;
provided at least one of A or B is -O-,
which comprises reacting a compound of the formula

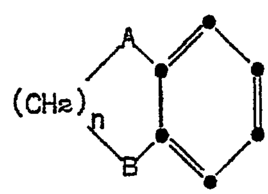

with a Villsmeier reagent of the formula

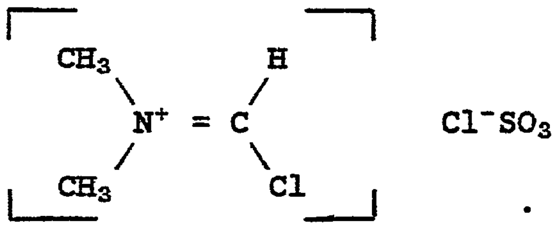

2. The process of claim 1 wherein the product is 1,3-benzodioxole-5-sulfonyl chloride.

3. The process of claim 1 wherein the product is dihydrobenzofuran-5-sulfonyl chloride.

4. The process of claim 1 wherein the product is 1,4-benzodioxan-6-sulfonyl chloride.

**Patentansprüche**

1. Verfahren zur Herstellung bizyklischer aromatischer Sulfonylchloride der Formel

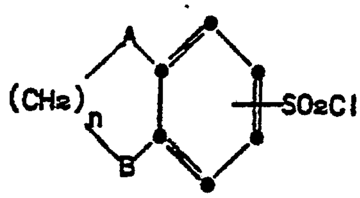

worin
n für 1 oder 2 steht,
A für –O– oder $-CH_2-$ steht und
B für –O–, $-CH_2-$ oder $=NCH_3$ steht,
mit der Maßangabe, daß wenigstens eine der Gruppen A oder B für –O– steht,
dadurch gekennzeichnet, daß eine Verbindung der Formel

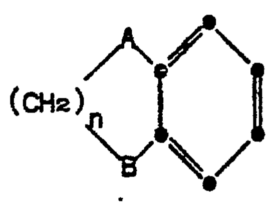

mit einem Villsmeier-Reagenz der Formel

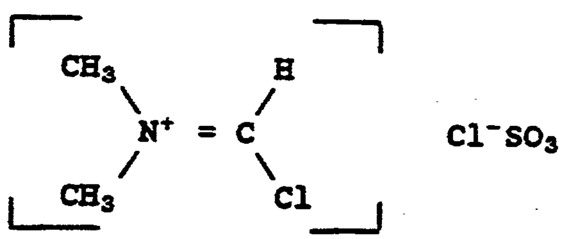

umgesetzt wird.

2. Verfahren nach Anspruch 1, dadurch gekennzeichnet, daß das Produkt 1,3-Benzodioxol-5-sulfonylchlorid ist.

3. Verfahren nach Anspruch 1, dadurch gekennzeichnet, daß das Produkt Dihydrobenzofuran-5-sulfonylchlorid ist.

4. Verfahren nach Anspruch 1, dadurch gekennzeichnet, daß das Produkt 1,4-Benzodioxan-6-sulfonylchlorid ist.

**Revendications**

1. Procédé de préparation de chlorures de sulfonyle bicycliques aromatiques de formule

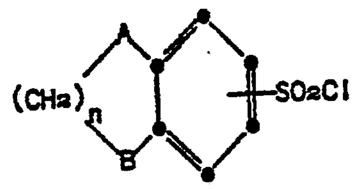

dans laquelle
n représente 1 ou 2;
A représente –O– ou $-CH_2$; et
B représente –O–, $-CH_2-$, ou $-NCH_3-$;
avec cette réserve qu'au moins un des radicaux A ou B représente –O–,
ce procédé consistant à faire réagir un composé de formule

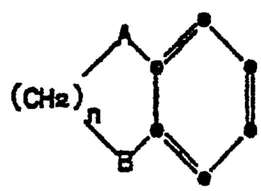

avec un réactif de Villsmeier de formule

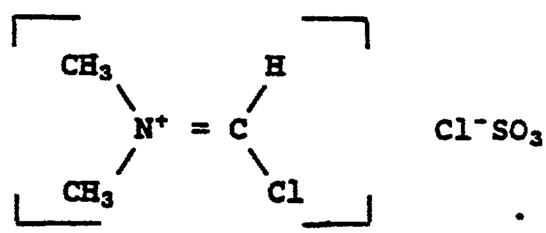

2. Procédé selon la revendication 1, caractérisé en ce que le produit est le chlorure de 1,3-benzodioxol-5-sulfonyle.

3. Procédé selon la revendication 1, caractérisé en ce que le produit est le chlorure de dihydrobenzofuranne-5-sulfonyle.

4. Procédé selon la revendication 1, caractérisé en ce que le produit est le chlorure de 1,4-benzodioxanne-6-sulfonyle.